(19) Europäisches Patentamt

European Patent Office

Office européen des brevets

(11) Veröffentlichungsnummer: **0 411 501 B1**

(12) **EUROPÄISCHE PATENTSCHRIFT**

(45) Veröffentlichungstag der Patentschrift: **14.09.94**

(21) Anmeldenummer: **90114473.3**

(22) Anmeldetag: **27.07.90**

(51) Int. Cl.5: **C12N 15/72**, C12P 21/02, C12N 15/12

(54) **Optimierte Fermentationsverfahren zur Herstellung von Fremdproteinen in E.coli.**

(30) Priorität: **02.08.89 DE 3925550**

(43) Veröffentlichungstag der Anmeldung:
**06.02.91 Patentblatt 91/06**

(45) Bekanntmachung des Hinweises auf die
Patenterteilung:
**14.09.94 Patentblatt 94/37**

(84) Benannte Vertragsstaaten:
**AT BE CH DE DK ES FR GB GR IT LI LU NL SE**

(56) Entgegenhaltungen:
**EP-A- 0 152 483**
**EP-A- 0 279 664**

**CHEMICAL ABSTRACTS, vol. 100, no. 7, 13 Februar 1984 Columbus, Ohio, USA CHOH-JI,T. et al.: "Effects of temperature shift on growth rate of Escherichia coli BB at lower glucose concentrarion."Seite 457; rechte Spalte; ref. no. 50036V.**

**GENE. vol. 69, no. 2, 1988, AMSTERDAM NL Seiten 301 - 315; AMANN, E. et al.:"Tightly regulated tac promoter vectors useful for the expression of unfused and fused proteins in Escherichia coli."**

(73) Patentinhaber: **BEHRINGWERKE Aktiengesellschaft**
**Postfach 1140**
**D-35001 Marburg (DE)**

(72) Erfinder: **Grote, Mathias, Dr.**
**Gladenbacher Weg 65**
**D-3550 Marburg (DE)**

# EP 0 411 501 B1

## Beschreibung

Die Erfindung beschreibt optimierte Fermentationsverfahren zur Herstellung von Fremdproteinen in E.coli unter Verwendung des lac-Promotors oder verbesserten lac-Promotors (z.B. tac, trc). Nach der Anwachsphase mit Kohlenstoffquelle Glukose erfolgt die Induktion der Produktbildung (1) über IPTG unter Glukose-Limitierung oder (2) über Laktose bzw. (3) über IPTG und Laktose unter Laktose-Limitierung. Die Limitierung von Glukose bzw. Laktose erfolgt so, daß der Sauerstoffpartialdruck oberhalb 10% bleibt.

Die Herstellung kommerzieller Mengen vieler verschiedener rekombinanter Proteine in E.coli ist im Prinzip gut bekannt. Die Expression dieser Proteine wird möglich, indem die kodierende cDNA in ein multicopy Plasmid mit den entsprechenden Sequenzen kloniert wird.

Übliche Expressionsversuche hierzu werden in Schüttelkolben durchgeführt. Die Ausbeute rekombinanter Proteine liegen hier in der Regel bei 50 - 100 mg/l, wenn Schüttelkolben-Kulturen mit Volumen von weniger als 100 ml eingesetzt werden.

Obwohl mit diesen Techniken erfolgreich rekombinante Proteine hergestellt werden können, besteht ein Bedarf an Techniken, mit denen die Proteinkonzentrationen und die herstellbaren Mengen deutlich erhöht werden. Ein Ansatz, der diese Forderungen erfüllt, ist die Entwicklung eines Fermentationsprozesses. Die Erfindung stellte sich folglich die Optimierung von Fermentationsverfahren zur Expression von Fremdproteinen in E.coli als Aufgabe.

In der Literatur wurden bereits mehrere derartige Prozesse und Verfahren beschrieben, mit denen die genannten Anforderungen zumindest teilweise erfüllt wurden. Mit dem hier verwendeten lac-Promotor wurden dabei meist Fusionsproteine mit einem N-terminalen $\beta$-Galaktosidaseanteil ($\beta$-Gal) hergestellt. Die Ausbeuten in der Fermentation liegen üblicherweise zwischen 0,1 bis 2,0 g Fusionsprotein pro Liter. Berücksichtigt man den Fusionsanteil von $\beta$-Gal, reduziert sich die eigentliche Produktkonzentration häufig auf 30% des genannten Wertes. Ferner sind aufwendige Reinigungsverfahren nötig, um den $\beta$-Gal-Anteil wieder vom Produkt zu trennen.

In der vorliegenden Erfindung wird u.a. die Expression eines reifen Produktes beschrieben, d.h. die Expression eines Produktes ohne Fusionsanteil, der später wieder abgespalten werden müßte. Derartige Verfahren ermöglichen eine relativ einfache Reinigung des Produktes. In der Fermentation sind jedoch sowohl die spezifischen als auch die volumetrischen Ausbeuten der Produkte normalerweise wesentlich geringer. Dies gilt insbesondere, wenn durch das Verfahren das Produkt in löslicher und biologisch voll aktiver Form hergestellt wird. Im Gegensatz zur Bildung von inaktivem und als "inclusion body's" abgelegtem Protein kann das lösliche und biologisch aktive Produkt in den Stoffwechsel der Zellen eingreifen und drastische Störungen im Organismus (E.coli) hervorrufen und kann wesentlich leichter von E. coli-Proteasen abgebaut werden. Ungeachtet dieser Problematik konnte in den bisherigen Verfahren, in dem Glukose als Kohlenstoffquelle und Isopropylthiogalaktosid (IPTG) zur Induktion eingesetzt wurden, Ausbeuten von 200 mg/l von biologisch voll aktivem Produkt erreicht werden.

Um die Fermentation zu optimieren, wurden erfindungsgemäße Verbesserungen des Wachstumsverhaltens und der Produktbildung unternommen. Da das Produkt intrazellulär gebildet wird, ist die spezifische Produktkonzentration (Produktmenge pro Zelle) und die Zellzahl von Bedeutung. Aus dem Produkt beider Faktoren ergibt sich die volumetrische Produktivität des Verfahrens in Gramm pro Liter(g/l).

In der Literatur sind häufig "high cell density fermentations" für rekombinante E.coli Stämme beschrieben. Hierbei werden in der Regel Zelldichten bis zu 30 g Trockensubstanz (TS) pro Liter (l) angegeben. Durch eine Kombination mehrerer im Prinzip bekannter Maßnahmen konnte ein Verfahren entwickelt werden, nach dem der rekombinante E.coli K12 Stamm bis zu Zelldichten von 50 g TS/l entsprechend 150 $A_{650}$ fermentiert wurde. Wesentlich hierbei ist, daß das nachfolgend beschriebene Verfahren keine Anreicherung der Zuluft mit Sauerstoff bedingt. Dies beeinflußt die Wirtschaftlichkeit des Verfahrens positiv, da der reine Sauerstoff als Substrat hohe Kosten verursacht und zusätzlich auf Maßnahmen zum Explosionsschutz verzichtet werden kann.

Ein wichtiger Faktor für ein Verfahren mit einer hohen volumetrischen Produktausbeute ist die optimale Induktion des Promotors. In der Literatur vielfach beschrieben ist die bei den vorher genannten niedrigen Zelldichten durchgeführte Induktion mit IPTG.

Es wurde gefunden, daß nach Induktion durch IPTG (1mM bis 10 mM, vorzugsweise 5 mM) und Limitierung von Glukose als Substrat dergestalt, daß der Sauerstoffpartialdruck größer/gleich 10% beträgt, eine Verbesserung der volumetrischen Ausbeuten um den Faktor 5 von 0,2 g/l auf 1,0 g/l erreicht wird.

Eine zweite Ausbildung der Erfindung besteht in der Induktion der Produktbildung bei Wachstum mit Laktose als Kohlenstoffquelle und gleichzeitig natürlichem Induktor. Durch Regelung der Laktosezugabe wurde ebenfalls wie oben der Sauerstoffpartialdruck größer/gleich 10% gehalten. Die Induktion durch Laktose wird in der Literatur als suboptimal angesehen, da diese Verfahrensweise im Vergleich zu IPTG-

EP 0 411 501 B1

Induktion angeblich einen geringeren Wirkungsgrad aufweist. Es sind bisher keine effizienten Fermentationsverfahren beschrieben worden, in denen Laktose als Induktor eingesetzt wurde. Der erfindungsgemäße Versuchsansatz beruht auf der Überlegung, daß durch eine schwächere Induktion und dadurch einer langsameren Produktbildung eine höhere Produktendkonzentration erreicht werden kann, da durch die langsamere Produktbildung der E.coli-eigene Stoffwechsel weniger störend beeinflußt wird. Dieser Überlegungsansatz konnte in entsprechenden Versuchen bestätigt werden, bei denen Produktkonzentrationen von 2 g/l ( +/-10%) entsprechend einer Verdopplung zu oben erreicht wurden. Das Verfahren unterscheidet sich von dem vorangegangenen IPTG-induzierten dadurch, daß in der linearen Wachstumsphase Glukose durch Laktose ersetzt wurden. Im Bereich hoher Stoffwechselaktivitäten am Ende der Fermentation, in dem auch Laktose limitiert zugegeben wurde, um den Partialdruck des Sauerstoffes oberhalb von 10% zu halten, konnte in einer dritten Verfahrensvariante die Induktion durch Laktose zusätzlich durch Zugaben von IPTG unterstützt werden. Diese zusätzliche IPTG Induktion ist apparatespezifisch nur dann erforderlich, wenn der Leistungseintrag des gewählten Fermenters nicht ausreichend ist, die Kultur mit Sauerstoff zu versorgen. Da in dem zweiten beschriebenen Verfahren ein erhöhter Plasmidverlust zu beobachten ist, ergibt sich im "scale up" ein Überschneidungspunkt bei Zunahme der Fermentationsvolumina, nach dem erst das zweite und dann das erste Verfahren wirtschaftlicher ist, da bei Induktion mit Laktose ein leicht erhöhter Plasmidverlust zu beobachten ist.

Zum Zeitpunkt der maximalen Produktkonzentration wird die Fermentation beendet. Die Biomasse wird mit für den Fachmann bekannten Verfahren aufkonzentriert (z.B. Separator) und aufgeschlossen (z.B. Hochdruckhomogenisator). Nach Sedimentation der Zellfragmente befindet sich der Hauptteil des Produktes im geklärten Überstand.

Die Erfindung betrifft demnach optimierte Fermentationsverfahren zur Expression von Fremdgenen in E.coli unter Kontrolle des lac-Promotors oder optimierten lac-Promotors, wobei die Induktion am Ende der exponentiellen Wachstumsphase durch

(1) IPTG bei gleichzeitiger substratlimitierter Glukosezugabe erfolgt, und durch die Limitierung der Glukosezugabe der Sauerstoffpartialdruck oberhalb von 10% gehalten wird,

(2) oder durch Laktose als Kohlenstoffquelle und gleichzeitig natürlichen Induktor erfolgt, wobei durch die Limitierung der Laktosezugabe der Sauerstoffpartialdruck oberhalb 10% gehalten wird,

(3) oder durch Laktose als Kohlenstoffquelle und gleichzeitig natürlichem Induktor sowie zusätzlich IPTG erfolgt, wobei durch die Limitierung der Laktosezugabe der Sauerstoffpartialdruck oberhalb 10% gehalten wird.

In bevorzugten Verfahrensvarianten wird jeweils der Sauerstoffpartialdruck erhöht durch eine oder mehrere nachfolgender Maßnahmen:

(a) Fermentation unter Überdruck, vorzugsweise bis zu 2 bar

(b) Geregeltes Nachverfahren des Leistungseintrags (Erhöhung der Rührerdrehzahl) und der Begasungsrate (bis zu 2 vvm)

(c) Absenken der Temperatur von 37°C auf bis zu 30°C um über einen verbesserten Henry-Koeffizienten und reduzierte Stoffwechselaktivität die Sauerstofftransferrate zu erhöhen und die Sauerstoffaufnahmerate zu reduzieren (notwendig im scale-up über 1 000 l, da spezifischer Leistungseintrag mit zunehmender Ansatzgröße ( = Behälter) sinkt.

Allen Verfahrensvarianten gemeinsam ist die geregelte Zugabe von Zuckersubstrat als Kohlenstoffquelle auf Werte von maximal 5 - 10 g/l und eine pH-Regulierung durch Zugabe von $NH_4 OH$ und $H_3 PO_4$ im Bereich von pH 6.7 bis pH 7.3 während der gesamten Fermentationsperiode.

Die vorstehend beschriebenen Verfahren werden vorzugsweise zur gentechnischen Herstellung der zu den Lipocortinen gehörenden Proteine PP4 und PP4-x (Grundmann et al., Proc. Natl. Acad. Sci. 85, (1985) 3708-3712) sowie deren Mutanten und Varianten eingesetzt.

Die Erfindung ist ferner in den Beispielen und Patentansprüchen beschrieben.

Beispiel:

In dem folgenden Beispiel wird die Fermentation von dem E.coli K12 Stamm W3110 lac IQ (Brent und Ptashne (1981) Proc.Acad.Natl.Sci. USA 78, 4204-4208) beschrieben, wobei dieser Stamm mit dem Plasmid pTrc99A-PP4 (Amann et al. (1988) Gene 69, 301-315) oder pTrc99A-PP4-X (Grundmann et al. (1988) Behring Inst. Mitt. 82, 59-67) transformiert war.

3

Ein gut geeignetes Medium ist in der Tab. 1 angegeben.

**Tab. 1**

**Zusammensetzung eines beispielhaften Wachstumsmediums;**
**(Angaben g bzw. mg pro Liter)**

| | | |
|---|---|---|
| Kohlenstoffquelle (Zucker) nach Bedarf | | |
| Hefeextrakt | 20 | g |
| $NaH_2PO_4$ x $H_2O$ | 1,2 | g |
| $Na_2HPO_4$ x 2 $H_2O$ | 8,5 | g |
| KCl | 1,0 | g |
| $MgSO_4$ x 7 $H_2O$ | 2,0 | g |
| Zitronensäure | 0,25 | g |
| $NH_4Cl$ | 5,0 | g |
| Thiamin | 5,0 | mg |
| $H_3BO_3$ | 2,0 | mg |
| $(NH_4)_6Mo_7O_{24}$x4 $H_2O$ | 0,8 | mg |
| Cu $SO_4$ x $5H_2$= | 0,16 | mg |
| KJ | 0,4 | mg |
| Mn $SO_4$ x 7 $H_2O$ | 2,02 | mg |
| $ZnSO_4$ x 7 $H_2O$ | 1,6 | mg |

Fermentiert wurde in einem 10 l Biostat E-Fermenter (Hersteller: Braun Melsungen) mit 8 l Fermentationsvolumen.

Während der Fermentation wurde kein Selektionsdruck auf plasmidhaltige Zellen ausgeübt, d.h. die Fermentation wurde ohne Zusatz von Antibiotika durchgeführt. Angeimpft wurde der Fermenter mit einer über-Nacht-Schüttelkolbenvorkultur. In der Anwachsphase wurde Glukose als Kohlenstoffquelle eingesetzt, wobei die Glukose zudosiert wurde, um in dieser Phase weniger als 0,1 M Essigsäure zu bilden. Erhöhte Acetatkonzentrationen führten zu signifikant niedrigeren Produktausbeuten. Nach 10-15 Stunden sind in der Anwachsphase Zellkonzentrationen von ca. 50 $OD_{650}$ erreicht worden und die Induktion der Produktbildung erfolgt alternativ auf drei verschiedene Arten:

(1) weitere Dosierung von Glukose nach Zugabe von 1-10 mM IPTG (vorzugsweise 5 mM IPTG)

Am Ende der Anwachsphase wurde die Produktbildung durch Zugabe von 1 10 m IPTG (vorzugsweise 5 mM IPTG) unter weiterer Dosierung von Glukose als Kohlenstoffquelle ("Substrat") induziert. Hierbei zeigte sich eine deutliche Abhängigkeit der Produktbildungsrate von der aktuellen Glukosekonzentration. Glukose als tatsächliches Substrat und IPTG als scheinbares Substrat treten als konkurrierende Substrate in Erscheinung, wobei nach den Regeln der Diauxie Glukose die Aktivierung des lac-Operons partiell bis vollständig unterdrückt. Im Glukose-limitierten System (Glukosekonzentration kleiner 0,1 g/l) wurden hier Ausbeuten von 1 g/l PP4 bzw. PP4-X erzielt.

Glukoselimitierung wurde durch Stellen der Pumpe oder mittels "on line"- HPLC Messung durchgeführt. Die Wachstumsrate der Zellen wurde durch Induktion in nicht-limitierten Systemen nicht reduziert, in limitierten Systemen reduziert sich die Wachstumsrate der Zellen in Abhängigkeit der Glukosekonzentration wie erwartet. In Abhängigkeit der entsprechenden Wachstumsraten wurden Zelldichten zwischen 100 und 150 $OD_{650}$ erreicht.

(2) weitere Dosierung von Laktose

Am Ende der Anwachsphase wurde die Produktbildung durch Ersetzen der Kohlenstoffquelle Glukose durch Laktose induziert. Laktose ist der physiologische Induktor des lac-Operons, bewirkt jedoch keine so vollständige Induktion wie IPTG. Während der Induktionsphase wuchsen die Zellen weiter auf Zelldichten von 100 $OD_{650}$. Die Produktkonzentration erreichte Werte von 1,5 g/l.

(3) weitere Dosierung von Laktose und Zugaben von 1-10 mM IPTG (vorzugsweise 5 mM)

Am Ende der Anwachsphase wurde die Produktbildung durch Ersetzen der Kohlenstoffquelle Glukose durch Laktose und zusätzliche Gabe von IPTG induziert. Hierbei wird die starke Induktion durch IPTG hervorgerufen und gleichzeitig das physiologische Substrat Laktose genutzt. Die exakte Dosierung der Kohlenstoffquelle ist hier im Gegensatz zur Glukose + IPTG nicht erforderlich. Ein Überschuß von Laktose bis zu 30 g/l beeinträchtigt die Produktivität nicht. Während der Induktion wachsen die Zellen ebenfalls weiter bis zu Zelldichten von 100 $OD_{650}$. Die Produktkonzentration am Ende der Fermentation beträgt 2,0 g/l.

In Abhängigkeit von der jeweiligen Fermentationsansatzgröße wird die Induktion nach einem der Verfahren durchgeführt werden, da die Plasmidstabilität von (1) zu (3) abnehmend ist.

Die in den beschriebenen Versuchen gewählten Fermentationsparameter sind in Tab. 2 zusammengefaßt.

Tab. 2

| Fermentationsparameter | |
|---|---|
| pH: | 7,0 (geregelt durch Zugabe von $H_3PO_4$ und $NH_4OH$) |
| Begasungsrate: | 0,5 - 2,0 vvm |
| Drehzahl: | 1 500 Rpm |
| Temperatur: | 37 °C (bis 30 °C) |
| Überdruck: | bis 2,0 bar |
| Substratkonzentration: | Glukose geregelt auf kleiner 5,0 g/l, limitiert bei Gelöstsauerstoffabnahme; Laktose geregelt in der Nachdosierung (kleiner 30 g/l), limitiert bei Gelöstsauerstoffabnahme. |
| Gelöstsauerstoff: | größer 10% |

**Patentansprüche**

1. Verfahren zur Expression Von Fremdgenen in E. coli unter Kontrolle des lac-Promotors oder optimierten lac-Promotors, dadurch gekennzeichnet, daß die Induktion am Ende der exponentiellen Wachstumsphase durch

   a) IPTG bei gleichzeitiger substratlimitierter Glucosezugabe erfolgt und durch die Limitierung der Glucosezugabe der Sauerstoffpartialdruck oberhalb von 10 % gehalten wird oder

   b) Lactose als Kohlenstoffquelle und gleichzeitig natürlichem Induktor erfolgt, wobei durch die Limitierung der Lactosezugabe der Sauerstoffpartialdruck oberhalb von 10 % gehalten wird oder

   c) Lactose als Kohlenstoffquelle und gleichzeitig natürlichem Induktor sowie zusätzlich IPTG erfolgt, wobei durch die Limitierung der Lactosezugabe der Sauerstoffpartialdruck oberhalb von 10 % gehalten wird.

2. Verfahren nach Anspruch 1, dadurch gekennzeichnet, daß mindestens einer der nachfolgenden Verfahrensbestandteile

   a) Fermentation unter Überdruck, vorzugsweise bis zu 2 bar,

   b) geregeltes Nachfahren des Leistungseintrags (Erhöhung der Rührerdrehzahl) und der Begasungsrate (bis zu 2 vvm),

   c) Absenken der Temperatur von 37 °C auf bis zu 30 °C,

   d) Geregelte Zugabe von Substrat als Kohlenstoffquelle auf Werte von maximal 5 bis 10 g/l

   e) pH Regelung auf Werte zwischen pH 6,7 bis pH 7,3

   zur Anwendung kommt.

**3.** Verfahren nach Anspruch 1, dadurch gekennzeichnet, daß E. coli-Stämme fermentiert werden, die cDNA von Lipocortinen enthalten.

**4.** Verfahren nach Anspruch 3, dadurch gekennzeichnet, daß die cDNA für PP4, PP4-x oder Mutanten und Varianten von PP4 kodiert.

**Claims**

**1.** A process for the expression of foreign genes in E.coli under the control of the lac promoter or optimized lac promotor, wherein induction is effected at the end of the exponential phase of growth by
a) IPTG with simultaneous substrate-limited glucose addition, and the oxygen partial pressure is maintained above 10 % by the limitation of the glucose addition, or
b) lactose as carbon source and simultaneously natural inducer, with the oxygen partial pressure being maintained above 10 % by the limitation of the lactose addition, or
c) lactose as carbon source and simultaneously natural inducer and, in addition, IPTG, with the oxygen partial pressure being maintained above 10 % by the limitation of the lactose addition.

**2.** The process as claimed in claim 1, wherein at least one of the following constituents of the process is applied
(a) fermentation under superatmospheric pressure, preferably up to 2 bar,
(b) controlled following of the power input (increasing the stirrer speed) and of the aeration rate (up to 2 vvm),
(c) reducing the temperature from 37°C to as far as 30°C,
(d) controlled addition of substrate as carbon source to maximum values of 5 to 10 g/l
(e) controlling the pH to values between pH 6.7 and pH 7.3.

**3.** The process as claimed in claim 1, wherein E.coli strains containing cDNA of lipocortins are fermented.

**4.** The process as claimed in claim 3, wherein the cDNA codes for PP4, PP4-x or mutants and variants of PP4.

**Revendications**

**1.** Procédé pour l'expression de gènes étrangers dans *E. coli,* sous le contrôle du promoteur *lac* ou de promoteurs *lac* optimisés, caractérisé en ce que l'induction s'effectue à la fin de la phase exponentielle de croissance
(1) par de l'IPTG avec addition simultanée et limitée de glucose c o m m e substrat, la pression partielle de l'oxygène étant maintenue au-dessus de 10 % par la limitation de l'addition de glucose, ou
(2) par du lactose en tant que source de carbone et en même temps inducteur naturel, la pression partielle de l'oxygène étant maintenue au-dessus de 10 % par la limitation de l'addition de lactose, ou
(3) par du lactose en tant que source de carbone et en même temps inducteur naturel, et en outre par de l'IPTG, la pression partielle de l'oxygène étant maintenue au-dessus de 10 % par la limitation de l'addition de lactose.

**2.** Procédé selon la revendication 1, caractérisé en ce que l'on utilise au moins l'une des composantes de processus suivantes:
a) fermentation sous une surpression, allant de préférence jusqu'à 2 bars,
b) intensification réglée de la puissance d'entraînement (augmentation du nombre de tours de l'agitateur) et du taux d'aération (jusqu'à 2 v/v/min),
c) abaissement de la température, de 37°C jusqu'à 30°C,
d) addition réglée du substrat, en tant que source de carbone, jusqu'à des valeurs de 5 à 10 g/l au maximum,
e) régulation du pH à des valeurs comprises entre pH 6,7 et pH 7,3.

**3.** Procédé selon la revendication 1, caractérisé en ce que l'on cultive par fermentation des souches de *E. coli* qui contiennent de l'ADNc de lipocortines.

4. Procédé selon la revendication 3, caractérisé en ce que l'ADNc code pour PP4, PP4-x ou des mutants et variants de PP4.